# EUROPEAN PATENT SPECIFICATION

(11) **EP 2 361 315 B1**
(45) Date of publication and mention of the grant of the patent: **05.03.2014**
(21) Application number: 09764773.9
(22) Date of filing: 25.11.2009
(51) Int. Cl.: A61K 39/395, A61K 47/42, C12Q 1/68

(54) **CXCL4L1 AS A BIOMARKER OF PANCREATIC CANCER**
CXCL4L1 ALS BIOMARKER FÜR PANKREASKARZINOM
CXCL4L1 EN TANT QUE BIOMARQUEUR DU CANCER DU PANCRÉAS

(30) Priority: 27.11.2008 EP 08305853
(43) Date of publication of application: 31.08.2011
(73) Proprietor: INSERM - Institut National de la Santé et de la Recherche Médicale, 75013 Paris (FR)
(72) Inventor: BIKFALVI, Andreas, F-33405 Talence (FR); PRATS, Hervé, 31 432 Toulouse Cedex 4 (FR); DUBRAC, Alexandre, F-33405 Talence (FR); HAGEDORN, Martin, F-33405 Talence (FR); DUMARTIN, Laurent, London EC1M 6BQ (GB)
(74) Representative: Cabinet Plasseraud
(86) International application number: PCT/EP2009/065805
(87) International publication number: WO 2010/060920

(56) References cited:
- WO-A-2006/029487
- VANDERCAPPELLEN J ET AL: "The role of CXC chemokines and their receptors in cancer" CANCER LETTERS, NEW YORK, NY, US, vol. 267, no. 2, 28 August 2008 (2008-08-28), pages 226-244, XP023181375 ISSN: 0304-3835 [retrieved on 2008-06-24]
- RAMAN ET AL: "Role of chemokines in tumor growth" CANCER LETTERS, NEW YORK, NY, US, vol. 256, no. 2, 20 September 2007 (2007-09-20), pages 137-165, XP022260861 ISSN: 0304-3835
- WENTE ET AL: "CXCL14 expression and potential function in pancreatic cancer" CANCER LETTERS, NEW YORK, NY, US, vol. 259, no. 2, 28 November 2007 (2007-11-28), pages 209-217, XP022392053 ISSN: 0304-3835
- SAUR ET AL: "CXCR4 Expression Increases Liver and Lung Metastasis in a Mouse Model of Pancreatic Cancer" GASTROENTEROLOGY, ELSEVIER, PHILADELPHIA, PA, vol. 129, no. 4, 1 October 2005 (2005-10-01), pages 1237-1250, XP005155709 ISSN: 0016-5085
- STRUYF SOFIE ET AL: "Platelet factor-4 variant chemokine CXCL4L1 inhibits melanoma and lung carcinoma growth and metastasis by preventing angiogenesis" CANCER RESEARCH, vol. 67, no. 12, June 2007 (2007-06), pages 5940-5948, XP002513222 ISSN: 0008-5472
- WENTE MORITZ N ET AL: "Expression and potential function of the CXC chemokine CXCL16 in pancreatic ductal adenocarcinoma" INTERNATIONAL JOURNAL OF ONCOLOGY, vol. 33, no. 2, August 2008 (2008-08), pages 297-308, XP009111632 ISSN: 1019-6439

## Description

### FIELD OF THE INVENTION:

The present invention relates to the use of CXCL4L1 as a biomarker of pancreatic cancer in a patient. The invention further relates to methods for the treatment of pancreatic cancer and/or the prevention of pancreatic metastasis.

### BACKGROUND OF THE INVENTION:

Pancreatic cancer is a malignant tumor of the pancreas. Each year about 35,000 individuals in the United States are diagnosed with this condition, and nearly the same number die from the disease. In Europe, more than 60,000 are diagnosed each year for this cancer. Thus, pancreatic cancer has one of the highest fatality rates of all cancers (Jemal et al., 2007) and therefore remains a major human health problem necessitating urgently an effective treatment for this mortal pathology.

Angiogenesis involves the formation of new blood vessels of capillary origin and this phenomenon is tightly controlled by a set of factors. It is now widely recognized that much of the angiogenic activity occurring in adults is pathological. Thus, angiogenesis and invasion are two closely linked processes playing an important role in disease progression such as cancer (Folkman, 1995). In particular, the expansion of solid tumors and other cancers critically depends on angiogenesis (Folkman, 1971) making anti-angiogenesis strategies relevant for cancer therapy (Folkman, 2001). On the other hand, invasion is required for local expansion of the tumor mass and for metastatic spread.

Amongst the investigated factors, there is CXCL4 which belongs to the CXC-chemokine family (ancient terminology PF4 for Platelet Factor 4) and which is synthetized not only in platelets or megacaryocytes but also in different other cell types including monocytes, T-cells, vascular smooth muscle cells and endothelial cells (Lasagni et al., 2007). CXCL4 or a peptide derived from its carboxyl-terminal domain (PF4/CTF) display significant antiangiogenic activity in vitro (Maione et al., 1990; Jouan et al., 1999; Hagedorn et al., 2001) and in vivo (Maione et al., 1990; Sharpe et al., 1990; Hagedorn et al., 2001). They suppress growth of various tumors (Tanaka et al., 1997; Maione et al., 1991) and metastasis (Kolber et al., 1995) in vivo. This effect is related to their antiangiogenic action and not to tumor cell proliferation (Sharpe et al., 1990; Tanaka et al., 1997; Maione et al., 1991; Kolber et al., 1995). Although CXCL4 is one of the first agents discovered to have an antiangiogenic action in ex vivo systems (Maione et al., 1990), the specific receptor mechanisms that transduce the antiangiogenic signal of CXCL4 are still poorly understood. Among other effects described of the molecule are notably immunomodulatory functions on T-cells (Romagnani et al., 2005).

Furthermore, a new homologous form of CXCL4 named CXCL4L1 has been identified in 1989 but only recently few data have been reported on the functional characterization of this chemokine. It is expressed in platelets but also in smooth muscle cells and endothelial cells. Recently the secretion mechanism of this molecule has been characterized in comparison to CXCL4 (Lasagni et al., 2007). CXCL4L1 is found in significant amounts in the medium of transfected HEK cells but not CXCL4L1. Furthermore, CXCL4L1 seems to be constitutively exported in a non-regulated manner whereas CXCL4 is exported in a regulated manner through its release from dense-core granules (DCG). This indicates that CXCL4L1 has a very different mechanism of export and diffusion from cells.

Also, CXCL4L1 was characterised as a potent inhibitor of angiogenesis more effective than CXCL4. For example, the international patent application WO 2006/029487 relates to CXCL4L1, fragments, and modified versions of CXCL4L1 and CXCL4L1 fragments for the prevention and/or reduction of angiogenesis, and more particularly for the treatment or prevention of angiogenic disorders or diseases involving angiogenic disorders or pathological angiogenesis such as cancer.

In parallel to this primordial therapeutic aspect for pancreatic cancer, an important need also relates to diagnostic of such a cancer.

Indeed, a major goal of the cancer biomarker field is the development of non-invasive tests that allow early cancer detection. Early detection of cancer is crucial for long-term survival and is particularly relevant to pancreatic cancer, which is a major cause of cancer death worldwide, with a five-year survival rate of less than 5%. Therefore, patients diagnosed with pancreatic cancer typically have a poor prognosis partly because the cancer usually causes no symptoms early on, leading to locally advanced or metastatic disease at time of diagnosis. Because pancreatic cancer is asymptomatic in the early stages, most patients are not diagnosed until the cancer has spread beyond the pancreas, which contributes significantly to the poor long-term survival rate as previously mentioned.

However, most solid tumors, including pancreatic cancer, can often be cured if they are detected and treated at an early stage.

Thus, there is still an existing need to develop a method for diagnosing pancreatic cancer, notably during early-stage of pancreatic cancer development.

### SUMMARY OF THE INVENTION:

The present invention relates to a method detecting a pancreatic cancer and/or pancreatic metastasis in a patient, said method comprising:
determining the expression level of the CXCL4L1 gene in a biological sample obtained from said patient,
   - comparing the expression level of CXCL4L1 gene with a predetermined threshold value,
wherein an elevated expression level of CXCL4L1 gene compared to said threshold value is indicative of pancreatic cancer.

The invention also relates to a method for staging a pancreatic cancer in a patient having pancreatic cancer comprising determining the expression level of the CXCL4L1 gene in a biological sample obtained from said patient, wherein the level of expression of the CXCL4L1 gene increases with the histological grade.

The invention also relates to the use of CXCL4L1 as a biomarker of pancreatic cancer in a patient.

The invention also relates to a CXCL4L1-specific binding molecule conjugated to an anti-cancer agent such as a cytotoxic agent or a growth inhibitory agent for use in the treatment of pancreatic cancer and/or the prevention of pancreatic metastasis, wherein said CXCL4L1-specific binding molecule is an anti-CXCL4L1 antibody or an aptamer.

### DETAILED DESCRIPTION OF THE INVENTION:

The inventors made the observation that CXCL4L1 may be used for an early and accurate detection of pancreatic cancer as well as a correlation between the expression level of CXCL4L1 gene and the severity of tumor grade.

### Definitions:

The term "CXCL4L1 ", as used herein, is intended to encompass all synonyms including, but not limited to, PFAv1, PF4var1, PF4ALT and SCYB4V1. The term thus includes naturally occurring CXCL4L1 and variants and modified forms thereof. The term "mature CXCL4L1 protein" refers to the mature CXCL4L1 protein of 70 amino acids which can be obtained by processing of a longer propeptide (provided in the GenPept database under accession number NP_002611) An exemplary native nucleotide sequence encoding for CXCL4L1 is provided in GenBank database under accession number NM_002620. It must be further noted that the term "CXCL4L1" also includes primate natural variants of the CXCL4 protein. By way of example, primate CXCL4L1 proteins are provided under GenBank accession number XM_001102971.1 (rhesus monkey) and XP_001156146.1 (chimpanzee).

As used herein, the term "CXCL4L1-specific binding molecule" is intended to refer to a molecule of sufficient size and complexity so as to be capable of selectively binding CXCL4L1.

The term "anti-CXCL4L1 antibody" refers to an antibody or a fragment thereof which selectively recognizes CXCL4L1.

As used herein, the term "pancreatic cancer" refers to pancreatic carcinomas notably pancreatic adenocarcinomas (e.g., pancreatic ductal adenocarcinomas) as well as other tumors of the exocrine pancreas (e.g., serous cystadenomas), acinar cell cancers, and pancreatic neuroendocrine tumors (such as insulinomas).

The term "pancreatic metastasis" has its general meaning in the art and refers to the spread of a tumor from pancreas to another non-adjacent organ or part.

Examples of pancreatic metastasis which express or over express CXCL4L1 and which could be treated with CXCL4L1 inhibitors include but are not limited to metastatic colon, lung, pancreas, oesophagus and prostate cancers, melanomas, hepatocarcinomas as well as ganglion or lymph node metastasis.

As used herein, the term "predetermined value" refers to the amount of CXCL4L1 in biological samples obtained from the general population or from a selected population of subjects. For example, the selected population may be comprised of apparently healthy subjects, such as individuals who have not previously had any sign or symptoms indicating the presence of pancreatic cancer. In another example, the predetermined value may be of the amount of CXCL4L1 obtained from subjects having an established pancreatic cancer. The predetermined value can be a threshold value or a range. The predetermined value can be established based upon comparative measurements between apparently healthy subjects and subjects with established pancreatic cancer.

The term "patient" or "subject" as used herein denote a monkey, a chimpanzee and a human. Preferably, a patient according to the invention is a human.

The term "healthy subjects" as used herein refers to a population of subjects who do not suffer from any known condition, and in particular, who are not affected with a pancreatic cancer.

The term "biological sample" means any biological sample derived from a patient. Examples of such samples include fluids, tissues, cell samples, organs, biopsies, etc. Preferred biological samples are a pancreatic tumor sample or biopsy. Preferred biological samples are whole blood, serum or plasma.

The term "biomarker", as used herein, refers generally to a molecule, i.e., a gene (or nucleic acid encoding said gene), protein, the expression of which in a biological sample from a patient can be detected by standard methods in the art (as well as those disclosed herein), and is predictive or denotes a condition of the patient from which it was obtained.

### Diagnostic methods and kits:

The present invention relates to a method for detecting pancreatic cancer and/or pancreatic metastasis in a patient comprising:
   - determining the expression level of the CXCL4L1 gene in a biological sample obtained from said patient,
   - comparing the expression level of CXCL4L1 gene with a predetermined threshold value,
wherein an elevated expression level of CXCL4L1 gene compared to said threshold value is indicative of pancreatic cancer..

The present invention also relates to method for staging a pancreatic cancer in a patient having pancreatic cancer comprising determining the expression level of the CXCL4L1 gene in a biological sample obtained from said patient, wherein the level of expression of the CXCL4L1 gene increases with the histological grade.

It is disclosed a method for monitoring a treatment of a patient affected by a pancreatic cancer and/or pancreatic metastasis with a CXCL4L1 antagonist comprising determining the expression level of the CXCL4L1 gene in a biological sample obtained from said patient, and optionally, comparing the expression level of the CXCL4L1 gene with a predetermined value representing a predetermined stage of the pancreatic cancer, the expression level of the CXCL4L1 gene with respect to the predetermined value indicating the evolution of the pancreatic cancer, and therefore the degree of efficacy of the treatment.

In a particular embodiment, the patient is affected with a pancreatic carcinoma such as a pancreatic ductal adenocarcinoma.

In one embodiment, determining the expression level of the CXCL4L1 gene according to methods of the invention is performed by determining the quantity of mRNA encoding CXCL4L1 in a biological sample obtained from said patient.

Pancreatic tumor sample is the preferred sample. Total RNAs can be easily extracted therefrom. The cell or tissue sample may be treated prior to its use, e.g. in order to render nucleic acids available. Techniques of cell or protein lysis, concentration or dilution of nucleic acids, are known by the skilled person.

Determination of the expression level of a gene can be performed by a variety of techniques. Generally, the expression level as determined is a relative expression level.

More preferably, the determination comprises contacting the sample with selective reagents such as probes, primers or ligands, and thereby detecting the presence, or measuring the amount of nucleic acids of interest originally in the sample.

In a preferred embodiment, the expression level may be determined by determining the quantity of mRNA.

Methods for determining the quantity of mRNA are well known in the art. For example the nucleic acid contained in the samples (e.g., biopsy prepared from the patient) is first extracted according to standard methods, for example using lytic enzymes or chemical solutions or extracted by nucleic-acid-binding resins following the manufacturer's instructions. The extracted mRNA is then detected by hybridization (e. g., Northern blot analysis) and/or amplification (e.g., RT-PCR). In a preferred embodiment, the expression level of the CXCL4L1 gene is determined by RT-PCR, preferably quantitative or semi-quantitative RT-PCR, even more preferably real-time quantitative or semi-quantitative RT-PCR.

Other methods of amplification include ligase chain reaction (LCR), transcription-mediated amplification (TMA), strand displacement amplification (SDA) and nucleic acid sequence based amplification (NASBA).

Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

The nucleic acid primers or probes used in the above amplification and detection method may be assembled as a kit. Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In another embodiment, determining the expression level of the CXCL4L1 gene according to methods of the invention is performed by measuring the concentration of the CXCL4L1 protein in a biological sample obtained from said patient.

In a preferred embodiment, the concentration of the CXCL4L1 protein is measured in a blood sample, a plasma sample or a serum sample obtained from said patient. Once the biological sample from the patient is prepared, the concentration of CXCL4L1 may be measured by any known method in the art.

In a particular embodiment, such methods comprise contacting the biological sample with a binding partner capable of selectively interacting with CXCL4L1 present in the biological sample. The binding partner may be an antibody that may be polyclonal or monoclonal, preferably monoclonal. In another embodiment, the binding partner may be an aptamer.

Polyclonal antibodies of the invention or a fragment thereof can be raised according to known methods by administering the appropriate antigen or epitope to a host animal selected, e.g., from pigs, cows, horses, rabbits, goats, sheep, and mice, among others. Various adjuvants known in the art can be used to enhance antibody production. Although antibodies useful in practicing the invention can be polyclonal, monoclonal antibodies are preferred.

Monoclonal antibodies of the invention or a fragment thereof can be prepared and isolated using any technique that provides for the production of antibody molecules by continuous cell lines in culture. Techniques for production and isolation include but are not limited to the hybridoma technique originally described by Kohler and Milstein (1975); the human B-cell hybridoma technique (Cote et al., 1983); and the EBV-hybridoma technique (Cole et al. 1985). For example, document TW 526269 discloses monoclonal antibodies against CXCL4L1.

Alternatively, techniques described for the production of single chain antibodies (see e.g. U.S. Pat. No. 4,946,778) can be adapted to produce anti- CXCL4L1, single chain antibodies. Antibodies useful in practicing the present invention also include anti-CXCL4L1 fragments including but not limited to F(ab')₂ fragments, which can be generated by pepsin digestion of an intact antibody molecule, and Fab fragments, which can be generated by reducing the disulfide bridges of the F(ab')₂ fragments. Alternatively, Fab and/or scFv expression libraries can be constructed to allow rapid identification of fragments having the desired specificity to CXCL4L1. For example, phage display of antibodies may be used. In such a method, single-chain Fv (scFv) or Fab fragments are expressed on the surface of a suitable bacteriophage, e. g., M13. Briefly, spleen cells of a suitable host, e. g., mouse, that has been immunized with a protein are removed. The coding regions of the VL and VH chains are obtained from those cells that are producing the desired antibody against the protein. These coding regions are then fused to a terminus of a phage sequence. Once the phage is inserted into a suitable carrier, e. g., bacteria, the phage displays the antibody fragment. Phage display of antibodies may also be provided by combinatorial methods known to those skilled in the art. Antibody fragments displayed by a phage may then be used as part of an immunoassay.

In another embodiment, the binding partner may be an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library, as described in Tuerk C. 1997. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consist of conformationally constrained antibody variable regions displayed by a platform protein, such as E. coli Thioredoxin A, that are selected from combinatorial libraries by two hybrid methods (Colas et al., 1996).

The binding partners of the invention such as antibodies or aptamers may be labelled with a detectable molecule or substance, such as a fluorescent molecule, an enzyme able to produce a coloured product, a radioactive molecule or any others labels known in the art. Labels are known in the art that generally provide (either directly or indirectly) a signal.

As used herein, the term "labelled", with regard to the antibody, is intended to encompass direct labelling of the antibody or aptamer by coupling (i.e., physically linking) a detectable substance, such as a radioactive agent or a fluorophore (e.g. fluorescein isothiocyanate (FITC) or phycoerythrin (PE) or indocyanine (Cy5), to the antibody or aptamer, as well as indirect labelling of the probe or antibody (e.g., horseradish peroxidise, HRP) by reactivity with a detectable substance. An antibody or aptamer of the invention may be labelled with a radioactive molecule by any method known in the art. For example radioactive molecules include but are not limited radioactive atom for scintigraphic studies such as 1123, 1124, In111, Re186, Re188.

The aforementioned assays generally involve the binding of the binding partner (i.e., antibody or aptamer) in a solid support. Solid supports which can be used in the practice of the invention include substrates such as nitrocellulose (e.g., in membrane or microtiter well form); polyvinylchloride (e.g., sheets or microtiter wells); polystyrene latex (e.g., beads or microtiter plates); polyvinylidine fluoride; diazotized paper; nylon membranes; activated beads, magnetically responsive beads, and the like.

More particularly, an ELISA method can be used, wherein the wells of a microtiter plate are coated with a set of anti-CXCL4L1 antibodies. A biological sample containing or suspected of containing CXCL4L1 is then added to the coated wells. After a period of incubation sufficient to allow the formation of antibody-antigen complexes, the plate(s) can be washed to remove unbound moieties and a detectably labelled secondary binding molecule added. The secondary binding molecule is allowed to react with any captured sample marker protein, the plate washed and the presence of the secondary binding molecule detected using methods well known in the art. An alternative method of ELISA involves the addition of a biological sample containing or suspected of containing CXCL4L1 to the coated wells in addition to a known amount of labelled CXCL4L1. After a period of incubation sufficient to allow the formation of antibody-antigen complexes and the competition between labelled and non-labelled naturally occurring molecules, the plate(s) can be washed to remove unbound moieties. The intensity of the label obtained by the binding of, for example HRP-conjugated CXCL4L1, is detected using methods well known in the art. In this configuration, that is more sensitive that common ELISA, decrease of the label is in relation with the amount of natural molecules present in the sample. In the case of HRP-conjugated CXCL4L1, the addition of a HRP substrate will allow the detection. Colour detection is commonly used but needs incubation time, whereas luminol-derived substances alternatives allow a direct (no incubation time) and a highly sensitive detection.

The concentration of CXCL4L1 may be measured by using standard immunodiagnostic techniques, including immunoassays such as competition, direct reaction, or sandwich type assays. Such assays include, but are not limited to, agglutination tests; enzyme-labelled and mediated immunoassays, such as ELISAs; biotin/avidin type assays; radioimmunoassays; immunoelectrophoresis; immunoprecipitation.

Measuring the concentration of CXCL4L1 (with or without immunoassay-based methods) may also include separation of the compounds: HPLC based on hydrophobicity; size exclusion chromatography based on size; and solid-phase affinity based on the compound's affinity for the particular solid-phase that is used. Once separated, CXCL4L1 may be identified based on the known "separation profile" e. g., retention time, for that compound and measured using standard techniques.

Alternatively, the separated compounds may be detected and measured by, for example, a mass spectrometer.

In one embodiment, the method of the invention further may comprise a step of comparing the concentration of CXCL4L1 with a predetermined threshold value. Said comparison is indicative of pancreatic cancer. CXCL4L1 is increased during the development of pancreatic cancer and notably during early-stage of pancreatic cancer development.

A further aspect of the invention relates to a method for detecting pancreatic cancer and/or pancreatic metastasis in a patient, said method comprising the steps of:
(i) measuring the concentration of CXCL4L1 in a biological sample obtained from said patient,
(ii) comparing the concentration of CXCL4L1 measured in step (i) to a reference value derived from the concentration of CXCL4L1 in a biological sample from a subject who does not suffer from pancreatic cancer, wherein an elevated level of CXCL4L1 in the biological sample obtained from said patient as compared to said reference value indicates that the patient suffers from a pancreatic cancer.

A further aspect of the invention relates to the use of CXCL4L1 as a biomarker of pancreatic cancer, in particular pancreatic adenocarcinoma, in a patient.

It is disclosed the use of a kit detecting CXCL4L1 for diagnosing pancreatic cancer in a patient.

### Therapeutic methods of the invention:

Another aspect of the invention relates to methods and compositions for the treatment of pancreatic cancer and/or the prevention of pancreatic metastasis.

Accordingly, the invention relates to a CXCL4L1-specific binding molecule that may be used as a targeting tumor agent to deliver radioisotopes, chemotherapy or other cytotoxic agents (i.e. TNFalpha) to the pancreatic tumour.

In a particular embodiment, the CXCL4L1 specific binding molecule is an aptamer as above described.

In another particular embodiment, the CXCL4L1 specific binding molecule is an antibody or and antibody fragment as above described.

According to the invention said CXCL4L1-specific binding molecule is conjugated to an anti-cancer agent such as a cytotoxic agent or a growth inhibitory agent.

A "growth inhibitory agent" when used herein refers to a compound or composition which inhibits growth of a cell, especially pancreatic cancer cell, either in vitro or in vivo. Examples of growth inhibitory agents include agents that block cell cycle progression, such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, and 5-fluorouracil. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

The term "cytotoxic agent" as used herein refers to a substance that inhibits or prevents the function of cells and/or causes destruction of cells. The term is intended to include radioactive isotopes (e.g. At211, I131, I125, Y90, Re186, Re188, Sm153, Bi212, P32, and radioactive isotopes of Lu), chemotherapeutic agents, e.g., methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, e.g., gelonin, ricin, saporin, and the various antitumor or anticancer agents disclosed below. Other cytotoxic agents are described below. A tumoricidal agent causes destruction of tumor cells.

Conjugation of the CXCL4L1-specific binding molecule of the invention with cytotoxic agents or growth inhibitory agents may be made using a variety of bifunctional protein coupling agents including but not limited to N-succinimidyl (2-pyridyldithio) propionate (SPDP), succinimidyl (N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6diisocyanate), and bis-active fluorine compounds (such as I,5-difluoro-2,4- dinitrobenzene). For example, a ricin immunotoxin can be prepared as described in Vitetta et al (1987). Carbon labeled 1-isothiocyanatobenzyl methyldiethylene triaminepentaacetic acid (MX-DTPA) is an exemplary chelating agent for conjugation of radionucleotide to the antibody (WO 94/11026).

The linker may be a "cleavable linker" facilitating release of the cytotoxic agent or growth inhibitory agent in the cell. For example, an acid-labile linker, peptidase-sensitive linker, photolabile linker, dimethyl linker or disulfide-containing linker (See e.g. U.S. Patent No. 5,208,020) may be used.

Alternatively, a fusion protein comprising the CXCL4L1-specific binding molecule of the invention (e.g. antibody) and cytotoxic agent or growth inhibitory agent may be made, by recombinant techniques or peptide synthesis. The length of DNA may comprise respective regions encoding the two portions of the conjugate either adjacent one another or separated by a region encoding a linker peptide which does not destroy the desired properties of the conjugate.

The CXCL4L1-specific binding molecule may be conjugated to a prodrug-activating enzyme which converts a prodrug (e.g. a peptidyl chemotlierapeutic agent, see WO81/01145) to an active anti-cancer drug (See, for example, WO 88/07378 and U.S. Patent No. 4,975,278). The enzyme includes any enzyme capable of acting on a prodrug in such a way so as to covert it into its more active, cytotoxic form. Enzymes that are useful in the method of this invention include, but are not limited to, alkaline phosphatase useful for converting phosphate-containing prodrugs into free drugs; arylsulfatase useful for converting sulfate-containing prodrugs into free drugs; cytosine deaminase useful for converting nontoxic fluorocytosine into the anticancer drug, 5-fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substituents; carbohydrate-cleaving enzymes such as O-galactosidase and neuraminidase useful for converting glycosylated prodrugs into free drugs; P-lactamase useful for converting drugs derivatized with P- lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. The enzymes can be covalently bound to the antibodies by techniques well known in the art such as the use of the heterobifunctional crosslinking reagents discussed above.

The invention also relates to a method for treating pancreatic cancer and/or preventing pancreatic metastasis which comprises the step of administering to a subject in need thereof a CXCL4L1-specific binding molecule as above described.

It is disclosed a CXCL4L1-specific binding molecule as above described for treating a patient affected with a pancreatic cancer, said patient being diagnosed as affected by a pancreatic cancer by the method as above described.

It is disclosed a CXCL4L1-specific binding molecule for treating a patient affected with a pancreatic cancer, wherein said patient has an expression level of the CXCL4L1 gene higher than a predetermined value obtained from the general population or from healthy subjects.

The CXCL4L1-specific binding molecule as above described may be administered in the form of a pharmaceutical composition.

Preferably, said inhibitor is administered in a therapeutically effective amount. By a "therapeutically effective amount" is meant a sufficient amount of said CXCL4L1-specific binding molecule to treat pancreatic cancer and/or to prevent pancreatic metastasis at a reasonable benefit/risk ratio applicable to any medical treatment.

It will be understood that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

The invention will further be illustrated in view of the following figures and examples.

### FIGURES:

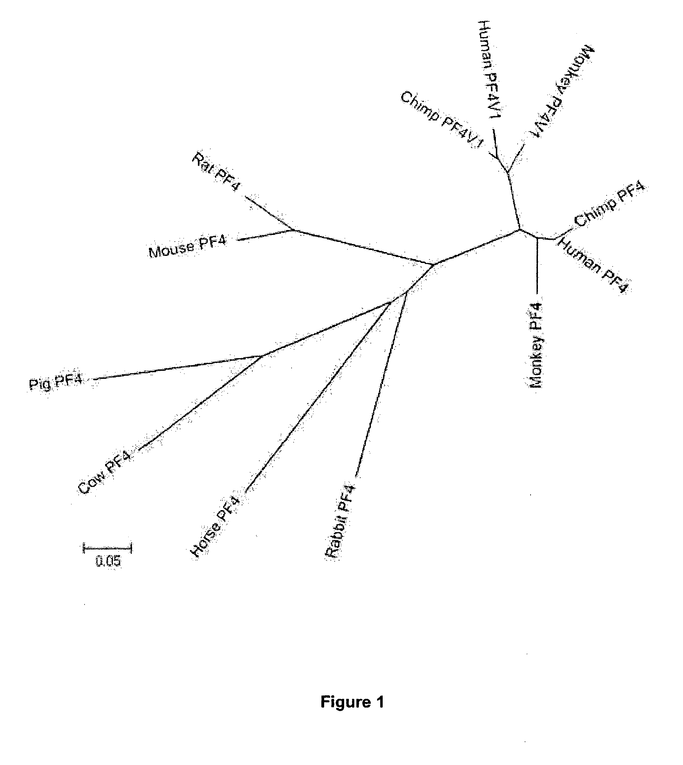

**Figure 1****:** Phylogenetic tree of CXCL4 or CXCL4L1. As seen in the figure, divergence occurred very late in evolution. CXCL4 of chimpanzee, monkey and human or CXCL4L1 from chimpanzee, monkey and human cluster together respectively.

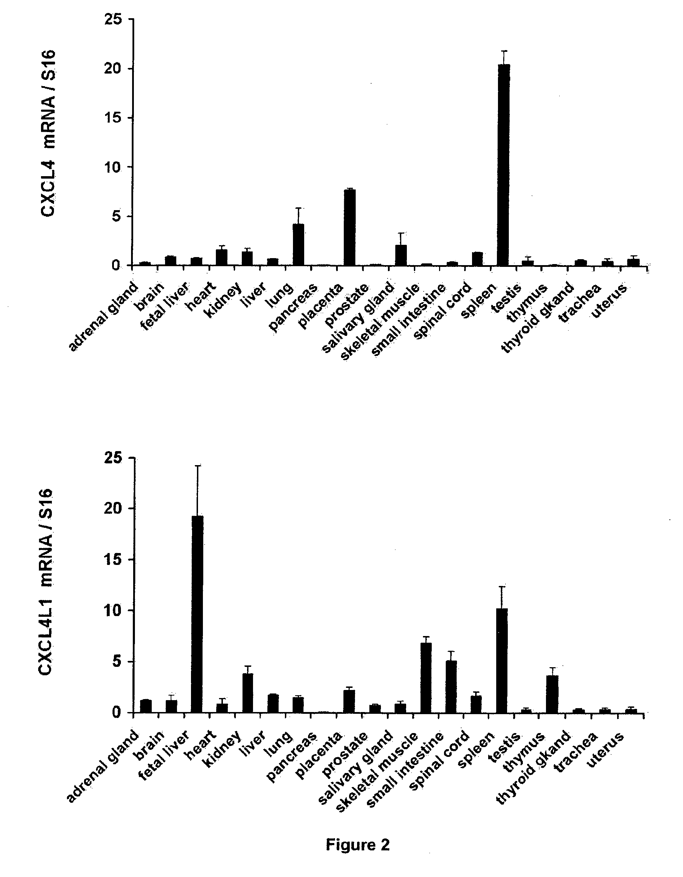

**Figure 2****:** Expression of CXCL4 and CXCL4L1 in different human tissues. Gene expressions were analyzed by semi-quantitative real-time PCR with specific primers for each gene and compared to housekeeping gene S16 expression.

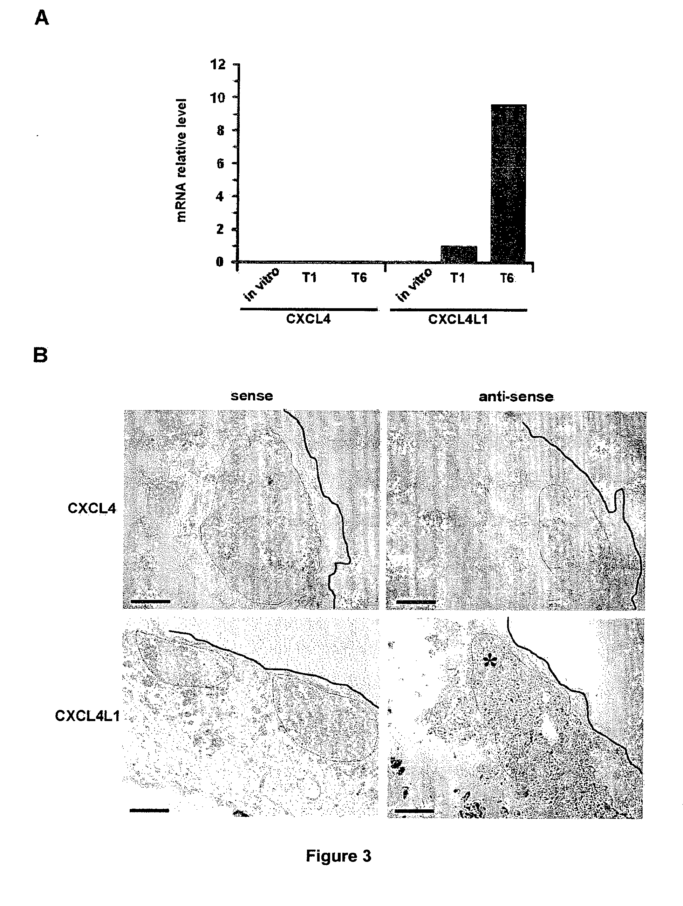

**Figure 3****:** mRNA expression analysis of CXCL4 and CXCL4L1 *in vitro* and *in vivo* in the PDAC-CAM model. **A.** Real-time PCR expression analysis in BxPC3 cells *in vitro* and in the PDAC-CAM model at T1 and T6, compared to S16 expression. CXCL4L1 mRNA relative level at T1 has been arbitrary given the value 1. **B.** *In situ* hybridization on paraffin-embedded sections of T6 PDAC-CAM samples with CXCL4L1 or CXCL4 riboprobes. Tumor nodes are delimited by dotted lines (asterix*: positive node) and the CAM surface by full lines. Scale bars 50µm.

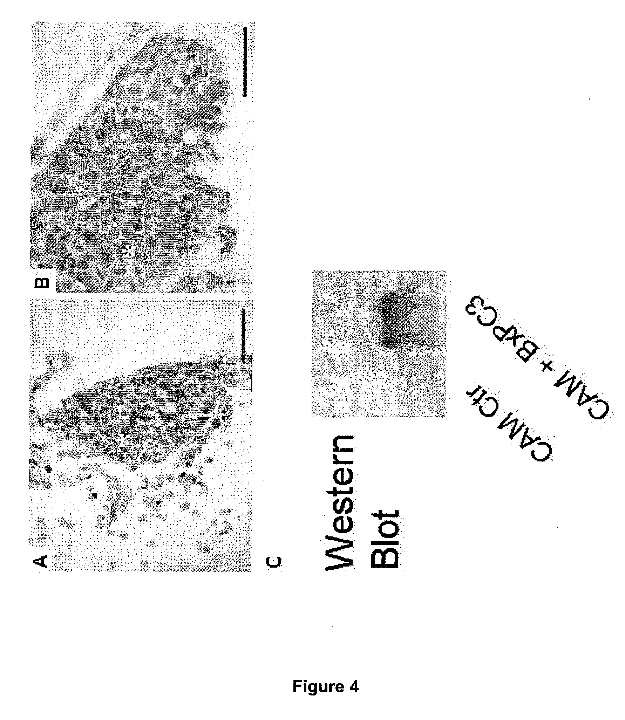

**Figure 4****:** Protein expression analysis of CXCL4L1 in the PDAC-CAM model. **A-B.** Immunolabelling on T6 paraffin-embedded sections with the pan CXCL4L1/CXCL4 antibody or the specific CXCL4 antibody respectively showing positive tumor nodes (asterix*). Scale bars 50µm. C. CAM tumor extracts were incubated with anti-CXCL4/CXCL4L1 antibody. Immunoprecipitates were then run on 10 % SDS polyacrylamide gel and Western-blot were performed after transfer using specific antiCXCL4L1 antibody. The gel is representative of two independent experiments.

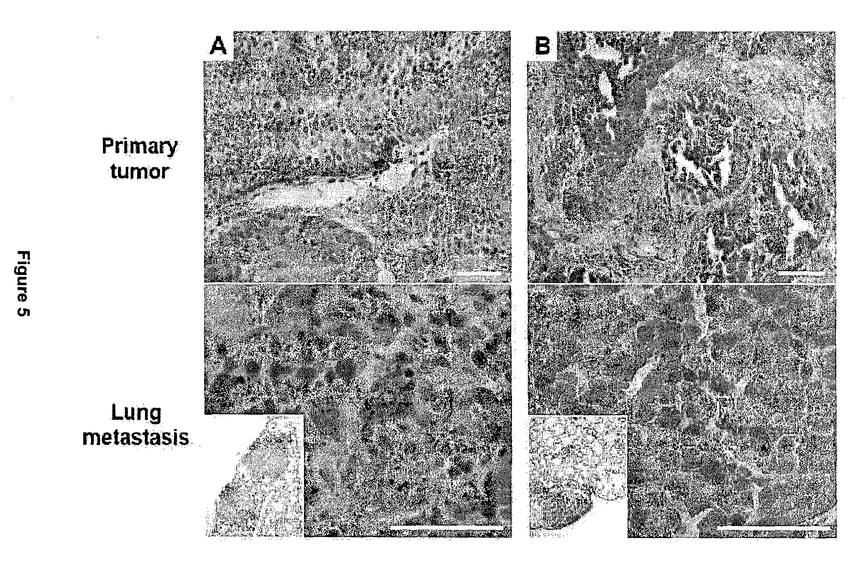

**Figure 5****:** Expression analysis of CXCL4L1 in a mouse xenograft model of pancreatic adenocarcinoma. Immunolabeling on paraffin-embedded sections of subcutaneous primary tumors **(A-B)** and lung micrometastasis **(C-D)** with the pan CXCL4L1/CXCL4 antibody **(A-C)** or the specific CXCL4 antibody **(B-D).** Inserts present global views of the respective micrometastases. Scale bars 50µm.

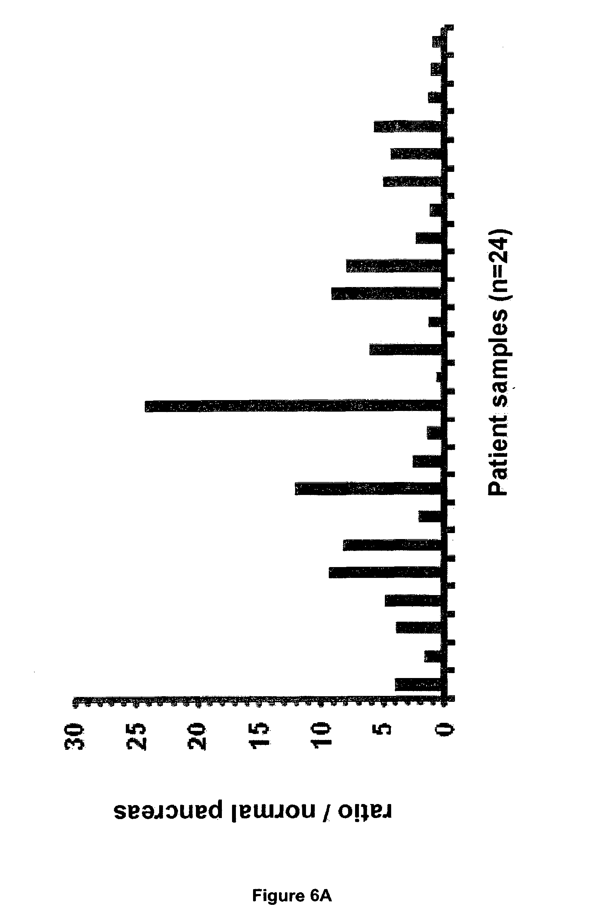

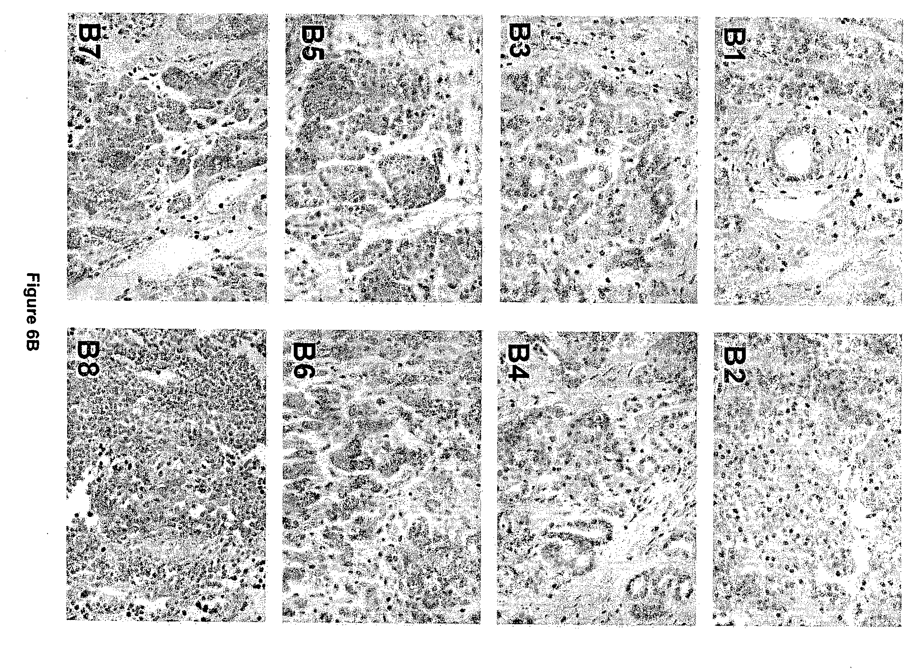

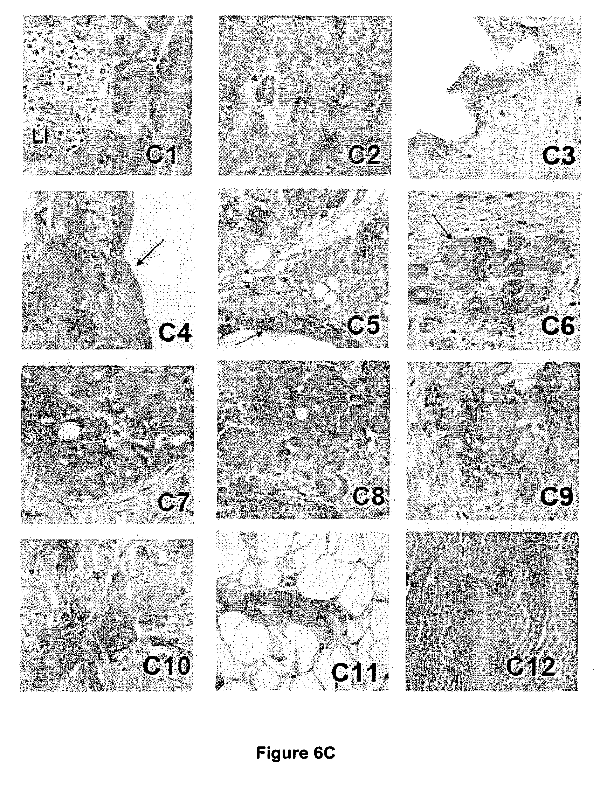

**Figure 6****:** Expression analysis of CXCL4L1 in human pancreatic tissues. **A.** Relative expression of CXCL4L1 in pancreatic adenocarcinoma samples of 24 human patients analyzed by real-time PCR. CXCL4L1 expression in human tumors is given in comparison with expression in normal pancreas. **B-C.** lmmunolabelling was done using either the polyclonal CXCL4/CXCL4L1 **(B)** or Mabv1 recognizing only CXCL4L1 (C). **B.** CXCL4/CXCL4L1 **B1-B2,** Control; Panel **B3-B7,** pancreatic adenocarcinoma at different stages; **B8,** ganglion metastasis. **C, C1-C3** weak background staining in normal pancreas, C4-9 strong immunoreactivity in pancreatic carcinoma, C10, positivity in invading cells, C11 invading cells in fat tissue, C12 positive lymph node metastasis.

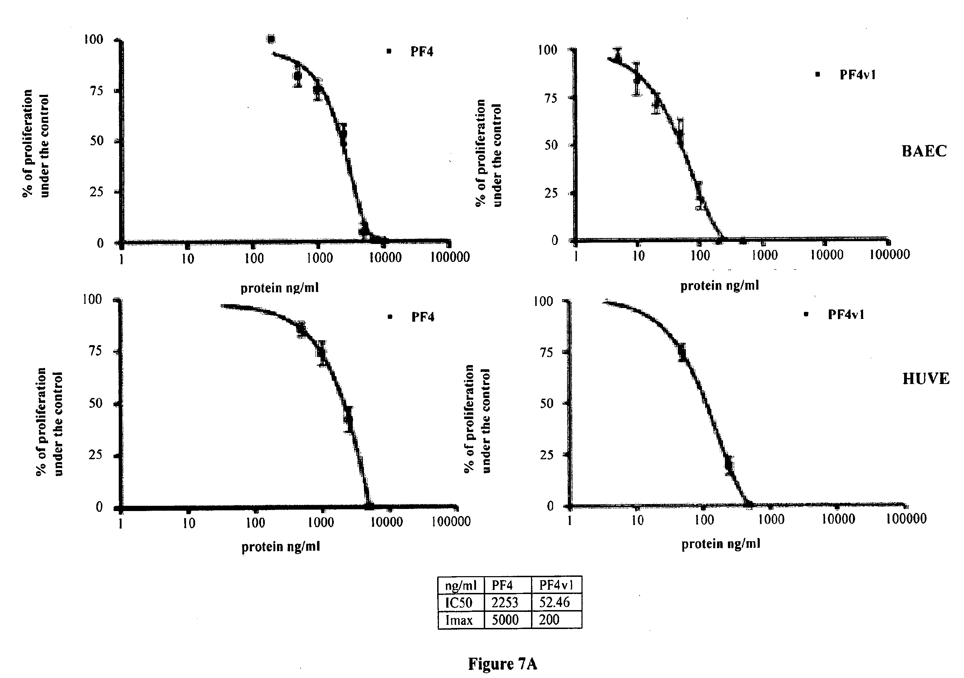

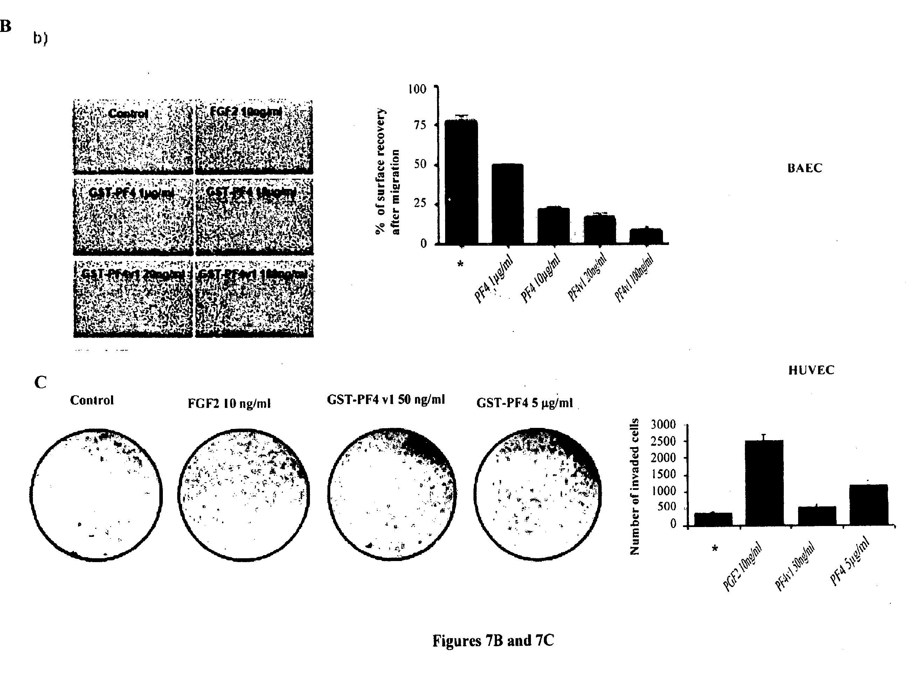

**Figure 7****:** Functional studies of CXCL4L1 on endothelial cells.
   **A-B.** Activity of recombinant GST-CXCL4 (GST-PF4) and GST-CXCL4L1 (GST-PF4v1) proteins on proliferation,migration and invasion of endothelial cells. BAEC and HUVEC cells were cultured in presence of FGF2 (10ng/ml) and different concentrations of recombinant proteins. **A.** After a 48h stimulation period, proliferation of cells was determined in comparison with cells uniquely treated with FGF2. IC50: protein concentration necessary to induce 50% of cell proliferation inhibition; Imax: protein concentration necessary for total inhibition of cell proliferation. **B.** After an 18h stimulation period, BAEC migration was determined by the percentage of covered surface from the initial wound. Results are average ± SEM of three independent experiments realized in triplicates. **C.** Effects of CXCL4L1 and CXCL4 on FGF-2-induced invasion of HUVECs. Left, images of invading cells; right, quantification.

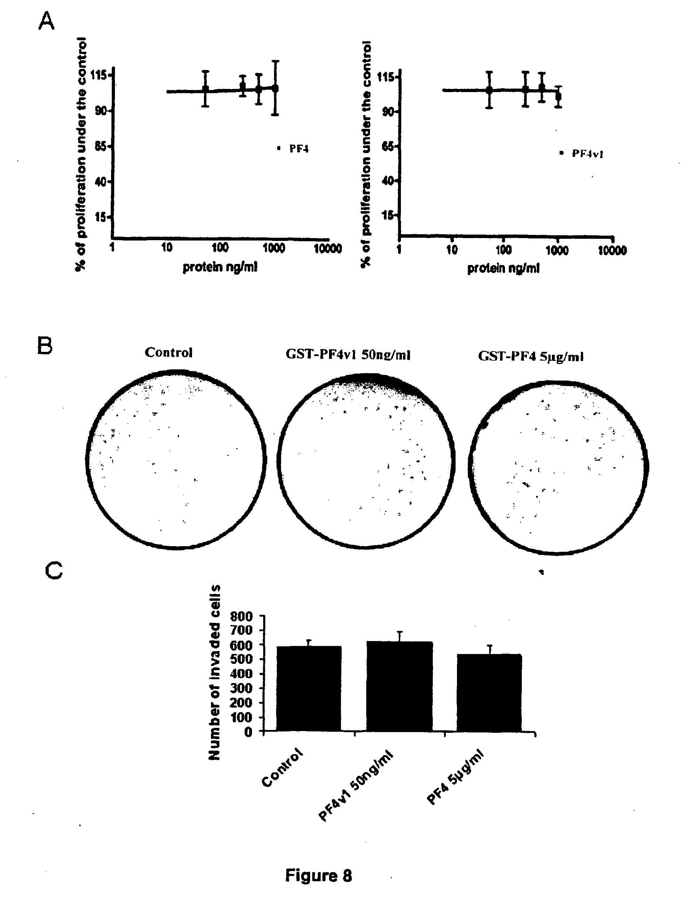

**Figure 8****:** Effect of CXCL4 and CXCL4L1 on pancreatic carcinoma cells (BXPC3)
   Effect of CXCL4L1 (PF4v1) and CXCL4 (PF4) on proliferation and invasion. A, effect on the proliferation of human ubilical veine endothelial cells (HUVEC). B, C Effect on invasion of HUVECs. B, images of invading cells, C, Quantification of invasion.

### EXAMPLE:

### Material & Methods

### Cell culture and PDAC-CAM model

Human pancreatic cancer cell lines BxPC3 and bovine aortic endothelial cells (BAEC) were cultured in DMEM 1 g/L glucose (Invitrogen, Cergy Pontoise, France) supplemented with 10% fetal bovine serum, antibiotics (penicillin/streptomycin) and L-glutamine. Human umbilical vein endothelial cells (HUVEC, Lonza, Levallois-Perret, France) were maintained in EBM-2 (Lonza) supplemented with EGM-2 SingleQuots (Lonza), which contain 2% FBS. Cultures were incubated at 37°C in 5% C02. Tumor cells were a kindly gift from Dr. C. Susini (INSERM U531, Toulouse, France). Fertilized chicken eggs (Gallus gallus) (EARL Morizeau, Dangers, France) were handled as previously described (Hagedorn et al., 2005). On embryonic day 10 (E10), 4 million BxPC3 cells diluted in serum free medium in a final volume of 40µl were deposited as a thin layer on the intact CAM surface.

### Mouse xenograft model

Female RAG-γ/c mice were housed and treated in the animal facility of Bordeaux 1 University ("Animalerie Mutualisée Bordeaux I"). All animal procedures were done according to institutional guidelines. Twenty weeks old mice (n=26) were anaesthetized with intraperitoneal injection of ketamine (150mg/kg) and xylazine (15mg/kg) and xenografted with 3.10⁶ BxPC3 cells in 100µl serum free medium by subcutaneous injection.

### RNA extraction and reverse transcription

Total RNA from cells or snap-frozen tissues were extracted by using RNeasy mini kit (Qiagen, Courtaboeuf, France). RNA quality and quantity were assessed by agarose gel electrophoresis and optical density measurement. First strand cDNA was prepared from 1µg of total RNA with Quantitect Reverse Transcription kit (Qiagen).

### Real-time PCR

Real-time PCR was carried out in a Mx3000P thermocycler (Stratagene, La Jolla, CA) by using SYBR Green dye (ABgene, Courtaboeuf, France). Human-specific primers were designed and evaluated for amplification efficiency using Universal Human Reference RNA (Stratagene). Only primers pairs with amplification efficacy between 90 and 110% were used. The PCR specificity was verified by dissociation curve analysis and agarose gel electrophoresis of the amplification product. All samples were tested in a minimum of three independent experiments.

### In Situ Hybridization

DIG CXCL4 and CXCL4L1 human riboprobes were synthesized according to the manufacturer's instructions (Roche RNA Labeling Kit (SP6/T7) - Roche). Briefly pCR2.1 TOPO CXCL4_UTRs and pCR2.1 TOPO CXCL4L1_UTRs vectors were linearized by Xhol (for sense probes) or BamHI (for anti-sense probes) enzyme digestion. After transcription, DNA templates were digested (DNasel - Invitrogen) and then, riboprobes were precipitated by lithium chloride.

CAMs with BxPC3 nodules were fixed overnight with PAF 4% at 4°C. After washing in PBS, tissues were dehydrated then, embedded in paraffin. Before hydridization, tissues sections were rehydrated, permeabilized by proteinase K and fixed again with PAF 4%. Paraffin-embedded CAM sections were hybridizated overnight at 70°C with CXCL4 or CXCL4L1 probes. For negative control, hybrydization with sense probes were performed. Tissues sections were incubated in sheep serum, washed and incubated with anti-DIG sheep antibody.

### Histology, immunohistochemistry

Paraffin-embedded tissues were cut into 7µm-thick microtome sections and stained with hematoxylin-eosin for histological analyses, localization of metastasis and selection of most representative tumor areas. Primary antibodies used were goat anti-human CXCL4 (PF4 - AF-795, R&D System - Minneapolis) and a mouse monoclonal antibody for CXCL4L1 (MabV1, clone 9E11-2D5-2G1). The production and characterization of this antibody will be reported elsewhere **(Dubrac et al manuscript in preparation).** Corresponding secondary antibodies were anti-goat or anti-mouse HRP-coupled antibodies (Dakocytomation). Imaging was carried out using a Nikon DXM Eclipse E600 microscope.

### Cell proliferation and cell death assays

Cell viability was evaluated by the WST-1 assay (Roche, Neuilly sur Seine Cedex, France). Relative abundance of apoptosis was measured by caspase 3/7 activity, according to manufacturer instructions (Apo-one homogeneous assay kit, Promega, Charbonnieres, France). In both cases, cells were seeded in 96-wells plates at a concentration of 1x10⁴ (HUVEC) or 3x10³ (BxPC3) cells/well and allowed to adhere overnight. Complete medium was replaced by serum-free medium with or without Gemcitabine (Laboratoire Lilly, Suresnes, France) and/or CXCL4L1 for 24 (HUVEC) or 48 (BxPC3) hours. All assays were performed in triplicate wells and each experiment was repeated three times.

### Migration and invasion assays

Cell migration and invasion assays were carried out using Transwell membrane filter with 8µm pore size (BD Biosciences, Le Pont-de-Claix, France) placed in 24-wells culture plates. The upper surface of the Transwell membrane was coated or not with 100µg/ml growth factor-reduced Matrigel matrix (BD Biosciences). Then, 1x10⁵ cells in serum-free medium were added to each Transwell chamber and allowed to migrate to the underside of the top chamber 6h for migration tests and 24h for invasion tests at 37°C with serum-free medium 0,5% FBS in the lower chamber as a chemoattractant. Cells were fixed 10 minutes with methanol 30%, acetic acid 10% and colored 3 minutes with coomassie blue 0,1%, methanol 30%, acetic acid 40%. Cells on the upper surface of the membrane were removed by wiping with a cotton swab.

### Phylogenetic analysis

A search for peptides matching CXCL4 (PF4) with BLAST against the Refseq database of proteins was performed and the peptide accessions pulled-out. Highlighted sequences designate the sequences that were successfully reciprocal BLAST back against human. Other sequences were listed for interest. A ClustalW program was run on the 12 sequences that were found representing CXCL4 (PF4) and CXCL4L1 (PF4V1) in all species.

The Phylogenetic tree was constructed using the neighbour joining method, using 1000 bootstraps to find best tree. Positions with gaps were excluded from the analysis and the alignment was corrected for multiple substitutions.

### Human tumor samples

Human adenocarcinoma samples were provided by Prof Martin Schilling (Klinik für Allgemeine Chirurgie, Viszeral-, Gefäß- und Kinderchirurgie, Homburg, Germany). Fresh tumor tissues were obtained during surgery and directly snap-frozen in liquid nitrogen. Patients gave their consent prior tissue analysis according to the clinics guidelines.

### Results

### Phylogenic analysis of CXCL4L1 and CXCL4

CXCL4L1 and CXCL4 are highly homologous. To analyze species expression of CXCL4L1 in comparison to CXCL4, we searched for peptides matching CXCL4/CXCL4L1 with BLAST against the Refseq database of proteins. CXCL4L1 is only expressed in man, monkey and chimpanzee. Of note, chicken does express neither CXCL4L1 nor CXCL4.

We have next performed a phylogenetic analysis using ClustalW program on the 12 sequences representing CXCL4 and CXCL4L1 in all species) and constructed a phylogenetic three using the neighbour joining method with 1000 bootstraps to find best tree (Figure 1). Positions with gaps were excluded from the analysis and the alignment was corrected for multiple substitutions. As seen from the analysis CXCL4L1 and CXCL4 diverged very late in evolution at the level of a common ancestor of monkeys, chimpanzees and humans.

### Expression of CXCL4L1 or CXCL4 in human tissues

Quantitative PCR in mRNA of several human tissues indicates different expression profiles for CXCL4L1 and CXCL4 (Figure 2). Whereas CXCL4L1 is merely expressed in the fetal liver, the colon, and to some extent in the spleen, CXCL4 is highly expressed in the spleen. Normal human pancreas does not express significantly of neither CXCL4L1 nor CXCL4.

### Expression of CXCL4L1 or CXCL4 in pancreatic adenocarcinoma

*Transcriptomic profiling identifies CXCL4L1 but not CXCL4 in the PDAC-CAM model:* Dual transcriptomic analysis using human Affymetrix or chicken Affymetrix microarrays have been performed between tumor day 1 (T1) and tumor day 6 (T6) of BxPC3 cells implantation into the chicken chorioallantoic membrane (corresponding to E11 and E16 of embryonic development). We clearly evidenced that CXCL4L1 but not CXCL4 was highly upregulated at T6 compared to T1 (14.6 fold increase). CXCL4L1 was also only detected in human Affymetrix array but no chicken ortholog was present when chicken chips were used. This indicated that CXCL4L1 is induced in tumor cells during in vivo tumor development.

*Expression analysis of CXCL4L1 or CXCL4 in the experimental pancreatic adenocarcinoma:* To reinforce this contention, we first undertook real-time PCR using specific primers for CXCL4L1 and CXCL4 to ascertain specific expression in the experimental pancreatic adenocarcinoma model. As seen with the transcriptomic analysis, qPCR analysis revealed high up-regulation in T6 tumors when compared to T1 tumors (9.65 fold). No expression was seen in BxPC3 cells in culture (Figure 3A).

We then investigated expression of CXCL4L1 by in situ hybridization in comparison to CXCL4 using specific probes recognizing either one of the chemokines (Figure 3B). In situ hybridization clearly demonstrated that only CXCL4L1 but not CXCL4 is expressed in T6 tumors.

We next investigated protein expression in our experimental pancreatic tumor samples of the PDAC-CAM model. Immunolocalization of CXCL4L1 using a specific polyclonal or monoclonal antibody that only recognizes human CXCL4 or CXCL4L1 clearly shows significant immunoreactivity in T6 tumors (Figure 4A) but not in BxPC3 cells (data not shown). In addition, a specific monoclonal anti-CXCL4L1 antibody (MABv1) clearly detects CXCL4L1 in pancreatic nodules in the CAM (Figure 4B).

Furthermore, we detected significant immunoreactivity of 7 kDa in western blotting using a monoclonal or polyclonal antibody able to interact with both CXCL4 and CXCL4L1. In addition, when using the specific monoclonal anti-CXCL4L1 antibody (MABv1) significant CXCL4L1 immunoreactivity was detected (Figure 4C).

Taken together, these data indicate that CXCL4L1 but not CXCL4 is highly overexpressed in pancreatic tumors that are grown on the chicken chorioallantoic membrane.

*Expression analysis in mouse models of pancreatic carcinoma:* To ascertain that the up-regulation of CXCL4L1 was not specific to the chicken embryo model, BxPC3 cells were first implanted subcutaneously in RAG- c mice and primary tumor or lung metastasis were analyzed 10 weeks after implantation. Tissues were then analyzed by qPCR or by immunohistochemistry using antibodies for human CXCL4L1 or CXCL4. qPCR analysis clearly indicates that CXCL4L1 but not CXCL4 is expressed in tumor samples. As seen in figure 5A, we next performed immunohistochemistry using the pan CXCL4L1/CXCL4 antibody that recognizes both forms. We clearly detected positive immunoreactivity in both, the primary tumor and lung metastasis. This antibody is specific for human CXCL4/CXCL4L1 but does not cross-react with mouse CXCL4. We next performed immunohistochemistry using our specific monoclonal antibody for CXCL4L1. Again, we clearly detected positive immunoreactivity in both, the primary tumor and lung metastasis (Figure 5B). These results indicate that CXCL4L1 is significantly expressed in this tumor model both in the primary tumor and in lung metastasis.

*Expression analysis of CXCL4*/*CXCL4L 1 in human tumor samples:* To ascertain expression of CXCL4L1 in pancreatic tumors, RNA was isolated from 24 adenocarcinoma patient samples and qPCR using specific primers were performed in comparison with normal pancreas (Figure 6A). CXCL4L1 mRNA expression was detected in all tested pancreatic tumors and overexpressed in 23/24 samples (average value +5.0) with various overexpression levels. We also performed qPCR with specific primers for CXCL4. No expression of CXCL4 could be detected in tumor samples from patients. These results indicate heterogeneous overexpression of CXCL4L1 in human pancreatic carcinoma which may reflect different patient subgroups.

We next undertook immunohistological analysis of pancreatic tumor samples using specific antibodies to human CXCL4/CXCL4L1 (Figure 6B and C). We clearly evidenced immunoreactivity in pancreatic tumor samples with both the polyclonal anti-CXCL4L/CXCL4L1 antibody (Figure 6B) or the specific monoclonal antibody MABv1 (Figure 6C) but not in normal human pancreas. Remarkably, immunoreactivity increased with severity of tumor grade, showing weakest staining in grade one tumors and strongest staining in grade 3 tumors. This indicates that CXCL4L1 is highly overexpressed in pancreatic adenocarcinoma and that expression increases with increasing histological grade.

**Functional studies with CXCL4L1 or CXCL4 in pancreatic carcinoma** CXCL4L1 and CXCL4 recombinant proteins were purified in significant amounts as GST-fusion proteins. Removal of GST by protease clipping had no significant effect on the biological activity making clipping unnecessary.

To test the activity of CXCL4L1 in comparison to CXCL4, BxPC3 cells or HUVEC cells were stimulated with increasing concentrations of these molecules and proliferation was measured. CXCL4L1 exhibited a much stronger effect on endothelial cell proliferation than CXCL4 (43 fold higher) similar as reported in the literature (Figure 7A). However, no effect of both chemokines was observed in BxPC3 cells (Figure 8). We further investigated the effect of CXCL4L1 or CXCL4 on migration and invasion using the Boyden chamber migration assay. Again, endothelial cell migration and invasion was significantly inhibited but not the migration of BxPC3 cells (Figure 7B, C; Figure 8 B,C).

### Discussion

CXCL4L1 is only expressed in human, monkey and chimpanzee and diverges from CXCL4 very late in evolution. Several human tissues expresses CXCL4L1 and CXCL4 including intestine, liver and spleen, but normal pancreatic tissue display weak or no expression of this chemokines. However, CXCL4L1 but not CXCL4 is significantly overexpressed in pancreatic carcinoma and represents a novel biomarker. This is based on the following observations:
(1) transcriptome analysis combined with qPCR or *in situ* hybridization indicates that human pancreatic adenocarcinoma cells when implanted in the chicken-chorioallantoic membrane express significant levels of CXCL4L1 mRNA but not of CXCL4;
(2) immunolocalization using anti-human CXCL4L1/CXCL4 or specific monoclonal anti-CXCL4L1 antibodies (Mabv1) evidences strong immunoreactivity in tumor cells when implanted in the chicken CAM;
(3) primary pancreatic tumors after implantation in mice express CXCL4L1 mRNA and primary tumor as well as metastatic tumor cells also exhibit positive immunoreactivity;
(4) pancreatic adenocarcinoma samples from human patient only express CXCL4L1 but not CXCL4 mRNA and exhibit strong positive immunoreactivity;
(5) endothelial cells but not pancreatic adenocarcinoma cells respond to exogenous CXCL4L1 indicating a paracrine mode of action of CXCL4L1 in pancreatic tumor development. Furthermore, our results indicate that upregulation of CXCL4L1 expression is due to a specific tumor-host interaction because pancreatic adenocarcinoma cells in culture fail to express either CXCL4L1 or CXCL4.

CXCL4L1 is a chemokine closely homologous to CXCL4 were only 34 % differences in the amino-terminus encoding the signal sequence and 4.3 % difference in the remaining sequence are observed. Platelet purified CXCL4L1 has been shown to be at least 50 times more potent than CXCL4 on cell migration (Stryuf et al. 2004). In our hands, recombinant CXCL4L1 is 50 times more potent than CXCL4 in inhibiting endothelial cell proliferation but 1000 times more potent than CXCL4 in inhibiting endothelial cell migration. Furthermore, inhibition of tumor development was observed in lung and melanoma carcinoma cells are inhibited by CXCL4L1 (Struyf et al., 2007)). In addition, there is previously shown that secretion and processing of CXCL4L1 and CXCL4 are different (Lasagni et al., 2007). Whereas CXCL4 is exported in a regulated manner through PKC-regulated pathway from dense-core-granules, CXCL4L1 is not regulated through PKC and constitutively secreted. Furthermore, CXCL4L1 or CXCL4 are not only expressed in the platelet-megacaryocytic lineage but also in inflammatory cells (T cells, leucocytes, monocytes), vascular and coronary smooth muscle cells and endothelial cells (Lasagni et al., 2007). These results indicate that CXCL4L1 may have regulatory functions in angiogenesis.

In the laboratory, we have initially developed a chicken embryo model to get better insights in glioma invasion and angiogenesis (Hagedorn et al., 2005). We have now further expanded this model for pancreatic ductal adenocarcinoma (PDAC-CAM model) and evidenced significant up-regulation of CXCL4L1, but not CXCL4 in pancreatic tumor cells when implanted on the CAM. Surprisingly, no CXCL4L1 expression was found in tumor cells in culture. Because specific primers for human CXCL4L1 were used and CXCL4L1 is only expressed in cells of human origin but not in other species, these results indicate that tumor cells are upregulating CXCL4L1 expression when placed in a suitable microenvironment. Furthermore, *in situ* hybridization using specific probes for CXCL4L1 or CXCL4 demonstrated a significant signal in tumors when grown in the CAM only for CXCL4L1. In addition, CXCL4L1 immunoreactivity was detected in tumor cells when implanted in the CAM but not when cells were grown in culture. These results also question the validity of some molecular profiling studies done in culture, because molecules of significant interest may not be detected under these conditions.

We next investigated CXCL4L1 expression using mice xenografted with pancreatic tumor cells. Subcutaneously implanted primary tumors expressed CXCL4L1 mRNA but not CXCL4 mRNA. Again high immunoreactivity was evidenced using anti-human CXCL4L1/CXCL4 or CXCL4L1 antibodies. Furthermore, significant immunoreactivity was also detected in lung metastasis. These results clearly indicate that upregulation is not specific to the chicken microenvironment but also detected when cells are implanted in the mouse tissue.

We finally, evidenced significant up-regulation of CXCL4L1 mRNA but not of CXCL4 mRNA in samples from human patients. mRNA expression was heterogeneous and probably indicates various patient groups with low, intermediate, or high CXCL4L1 expression. An interesting finding we observed was that immunoreactivity for CXCL4L1 increased with histological grade. Significant immunoreactivity was also detected in lymph node metastatic lesions. This indicates that CXCL4L1 constitutes a novel biomarker for pancreatic carcinoma and predicts evolution of the disease and treatment response.

Furthermore, due to a local increase in the concentration of CXCL4L1 in the tumor environment, pancreatic tumors can be targeted by a CXCL4L1-specific binding molecule conjugated to an anti-cancer agent such as a cytotoxic agent or a growth inhibitory agent for therapeutic purposes.

### REFERENCES:

Brummelkamp TR, Bernards R, Agami R. A system for stable expression of short interfering RNAs in mammalian cells. Science. 2002 Apr 19;296(5567):550-3.
Colas P, Cohen B, Jessen T, Grishina I, McCoy J, Brent R. (1996) Genetic selection of peptide aptamers that recognize and inhibit cyclin-dependent kinase 2. Nature, 380, 548-50.
Cote RJ, Morrissey DM, Houghton AN, Beattie EJ Jr, Oettgen HF, Old LJ. Generation of human monoclonal antibodies reactive with cellular antigens. Proc Natl Acad Sci U S A. 1983 Apr;80(7):2026-30.
Elbashir SM, Harborth J, Lendeckel W, Yalcin A, Weber K, Tuschl T. Duplexes of 21-nucleotide RNAs mediate RNA interference in cultured mammalian cells. Nature. 2001 May 24;411 (6836):494-8.
Folkman J: Angiogenesis in cancer, vascular, rheumatoid and other disease, Nat Med 1995, 1:27-31
Folkman J: Tumor angiogenesis: therapeutic implications, N Engl J Med 1971, 285:1182-1186
Folkman J: Angiogenesis-dependent diseases, Semin Oncol 2001, 28:536-542
Maione TE, Gray GS, Petro J, Hunt AJ, Donner AL, Bauer SI, Carson HF, Sharpe RJ: Inhibition of angiogenesis by recombinant human platelet factor-4 and related peptides, Science 1990, 247:77-79
Hagedorn M, Zilberberg L, Lozano RM, Cuevas P, Canron X, Redondo-Horcajo M, Gimenez-Gallego G, Bikfalvi A: A short peptide domain of platelet factor 4 blocks angiogenic key events induced by FGF-2, Faseb J 2001, 15:550-552
Hagedorn M, Javerzat S, Gilges D, Meyre A, de Lafarge B, Eichmann A, Bikfalvi A: Accessing key steps of human tumor progression in vivo by using an avian embryo model, Proc Natl Acad Sci U S A 2005, 102:1643-1648
Hannon GJ. RNA interference. Nature. 2002 Jul 11;418(6894):244-51.
Jemal A, Siegel R, Ward E, Murray T, Xu J, Thun MJ (2007). "Cancer statistics, 2007". CA Cancer J Clin 57 (1): 43-66.
Jouan V, Canron X, Alemany M, Caen JP, Quentin G, Plouet J, Bikfalvi A: Inhibition of in vitro angiogenesis by platelet factor-4-derived peptides and mechanism of action, Blood 1999, 94:984-993
Kolber DL, Knisely TL, Maione TE: Inhibition of development of murine melanoma lung metastases by systemic administration of recombinant platelet factor 4, J Natl Cancer Inst 1995, 87:304-309
Kohler G, Milstein C. Continuous cultures of fused cells secreting antibody of predefined specificity. Nature. 1975 Aug 7;256(5517):495-7.
Lasagni L, Grepin R, Mazzinghi B, Lazzeri E, Meini C, Sagrinati C, Liotta F, Frosali F, Ronconi E, Alain-Courtois N, Ballerini L, Netti GS, Maggi E, Annunziato F, Serio M, Romagnani S, Bikfalvi A, Romagnani P: PF-4/CXCL4 and CXCL4L1 exhibit distinct subcellular localization and a differentially regulated mechanism of secretion, Blood 2007, 109:4127-4134
McManus MT, Sharp PA. Gene silencing in mammals by small interfering RNAs. Nat Rev Genet. 2002 Oct;3(10):737-47.
Maione TE, Gray GS, Hunt AJ, Sharpe RJ: Inhibition of tumor growth in mice by an analogue of platelet factor 4 that lacks affinity for heparin and retains potent angiostatic activity, Cancer Res 1991, 51:2077-2083
Romagnani P, Maggi L, Mazzinghi B, Cosmi L, Lasagni L, Liotta F, Lazzeri E, Angeli R, Rotondi M, Fili L, Parronchi P, Serio M, Maggi E, Romagnani S, Annunziato F: CXCR3-mediated opposite effects of CXCL10 and CXCL4 on TH1 or TH2 cytokine production, J Allergy Clin Immunol 2005, 116:1372-1379
Sharpe RJ, Byers HR, Scott CF, Bauer SI, Maione TE: Growth inhibition of murine melanoma and human colon carcinoma by recombinant human platelet factor 4, J Natl Cancer Inst 1990, 82:848-853
Struyf S, Burdick MD, Proost P, Van Damme J, Strieter RM: Platelets release CXCL4L1, a nonallelic variant of the chemokine platelet factor-4/CXCL4 and potent inhibitor of angiogenesis, Circ Res 2004, 95:855-857
Struyf S, Burdick MD, Peeters E, Van den Broeck K, Dillen C, Proost P, Van Damme J, Strieter RM.Platelet factor-4 variant chemokine CXCL4L1 inhibits melanoma and lung carcinoma growth and metastasis by preventing angiogenesis. Cancer Res. 2007 Jun 15;67(12):5940-8.
Tanaka T, Manome Y, Wen P, Kufe DW, Fine HA: Viral vector-mediated transduction of a modified platelet factor 4 cDNA inhibits angiogenesis and tumor growth, Nat Med 1997, 3:437-442
Tuerk C., Using the SELEX combinatorial chemistry process to find high affinity nucleic acid ligands to target molecules. Methods Mol Biol. 1997; 67: 219-30.
Tuschl T, Zamore PD, Lehmann R, Bartel DP, Sharp PA. Targeted mRNA degradation by double-stranded RNA in vitro. Genes Dev. 1999 Dec 15; 13(24):3191-7.

## Claims

1. A method for detecting a pancreatic cancer and/or pancreatic metastasis in a patient comprising :
- determining the expression level of the CXCL4L1 gene in a biological sample obtained from said patient,
- comparing the expression level of CXCL4L1 gene with a predetermined threshold value,
wherein an elevated expression level of CXCL4L1 gene compared to said threshold value is indicative of pancreatic cancer.

2. A method for staging a pancreatic cancer in a patient having pancreatic cancer comprising :
- determining the expression level of the CXCL4L1 gene in a biological sample obtained from said patient,
wherein the level of expression of the CXCL4L1 gene increases with the histological grade.

3. The method according to claim 1 or 2 wherein said expression level of the CXCL4L1 gene is determined by measuring the quantity of the CXCL4L1 mRNA and said biological sample is a tissue sample.

4. The method according to claim 3wherein said tissue sample is a pancreatic tumor sample.

5. The method according to claim 1 or2 wherein said expression level of the CXCL4L1 gene is determined by measuring the concentration of the CXCL4L1 protein in a biological sample obtained from said patient.

6. The method according to claim 5 wherein said biological sample is a blood sample, a serum sample or a plasma sample.

7. The method according to any one of claims 1 to 6 wherein said pancreatic cancer is a pancreatic adenocarcinoma.

8. Use of CXCL4L1 as a biomarker of pancreatic cancer in a patient.

9. A CXCL4L1-specific binding molecule conjugated to an anti-cancer agent such as a cytotoxic agent or a growth inhibitory agent for use in the treatment of pancreatic cancer and/or the prevention of pancreatic metastasis, wherein said CXCL4L1-specific binding molecule is an anti-CXCL4L1 antibody or an aptamer.

10. The CXCL4L1-specific binding molecule for use in the treatment of pancreatic cancer and/or the prevention of pancreatic metastasis according to claim 9 wherein said CXCL4L1-specific binding molecule is an anti-CXCL4L1 antibody.

## Patentansprüche

1. Verfahren zum Nachweis von Bauchspeicheldrüsenkrebs und/oder Bauchspeicheldrüsenmetastasen in einem Patienten, umfassend:
- die Bestimmung des Expressionsspiegels des CXCL4L1-Gens in einer biologischen Probe, die von dem Patienten erhalten wurde,
- das Vergleichen des Expressionsspiegels des CXCL4L1-Gens
mit einem vorgegebenen Schwellenwert,
wobei ein erhöhter Expressionsspiegel des CXCL4L1-Gens, verglichen mit dem Schwellenwert, auf Bauchspeicheldrüsenkrebs schließen lässt.

2. Verfahren zur Bestimmung des Stadiums von Bauchspeicheldrüsenkrebs in einem Patienten, der Bauchspeicheldrüsenkrebs hat, umfassend:
- die Bestimmung des Expressionsspiegels des CXCL4L1-Gens in einer biologischen Probe, die von dem Patienten erhalten wurde,
wobei der Expressionsspiegel des CXCL4L1-Gens mit dem histologischen Grad steigt.

3. Verfahren nach Anspruch 1 oder 2, wobei der Expressionsspiegel des CXCL4L1-Gens durch Messung der Menge an CXCL4L1-mRNA bestimmt wird und die biologische Probe eine Gewebeprobe ist.

4. Verfahren nach Anspruch 3, wobei die Gewebeprobe eine Tumorprobe der Bauchspeicheldrüse ist.

5. Verfahren nach Anspruch 1 oder 2, wobei der Expressionsspiegel des CXCL4L1-Gens durch Messung der Konzentration des CXCL4L1-Proteins in einer biologischen Probe, die von dem Patienten erhalten wurde, bestimmt wird.

6. Verfahren nach Anspruch 5, wobei die biologische Probe eine Blutprobe, eine Serumprobe oder eine Plasmaprobe ist.

7. Verfahren nach einem der Ansprüche 1 bis 6, wobei der Bauchspeicheldrüsenkrebs Adenokarzinom der Bauchspeicheldrüse ist.

8. Verwendung von CXCL4L1 als Biomarker für Bauchspeicheldrüsenkrebs in einem Patienten.

9. CXCL4L1-spezifisches Bindemolekül, das verbunden ist mit einem anti-Krebs-Wirkstoff, wie beispielsweise einem zytotoxischen Wirkstoff oder einem wachstumshemmenden Wirkstoff, zur Verwendung bei der Behandlung von Bauchspeicheldrüsenkrebs und/oder der Vorbeugung von Bauchspeicheldrüsenmetastasen, wobei das CXCL4L1-spezifische Bindemolekül ein anti-CXCL4L1-Antikörper oder ein Aptamer ist.

10. CXCL4L1-spezifisches Bindemolekül zur Verwendung bei der Behandlung von Bauchspeicheldrüsenkrebs und/oder der Vorbeugung von Bauchspeicheldrüsenmetastasen nach Anspruch 9, wobei das CXCL4L1-spezifische Bindemolekül ein anti-CXCL4L1-Antikörper ist.

## Revendications

1. Procédé de détection d'un cancer du pancréas et/ou de métastases pancréatiques chez un patient, comprenant :
- la détermination du niveau d'expression du gène CXCL4L1 dans un échantillon biologique obtenu dudit patient,
- la comparaison du niveau d'expression du gène CXCL4L1 avec une valeur seuil prédéterminée,
dans lequel un niveau d'expression élevé du gène CXCL4L1 par rapport à ladite valeur seuil est indicatif d'un cancer du pancréas.

2. Procédé de détermination du stade d'un cancer du pancréas chez un patient ayant un cancer du pancréas, comprenant :
- la détermination du niveau d'expression du gène CXCL4L1 dans un échantillon biologique obtenu dudit patient,
dans lequel le niveau d'expression du gène CXCL4L1 augmente avec le grade histologique.

3. Procédé selon la revendication 1 ou 2, dans lequel ledit niveau d'expression du gène CXCL4L1 est déterminé en mesurant la quantité d'ARNm de CXCL4L1 et ledit échantillon biologique est un échantillon de tissu.

4. Procédé selon la revendication 3, dans lequel ledit échantillon de tissu est un échantillon de tumeur du pancréas.

5. Procédé selon la revendication 1 ou 2, dans lequel ledit niveau d'expression du gène CXCL4L1 est déterminé en mesurant la concentration de la protéine CXCL4L1 dans un échantillon biologique obtenu dudit patient.

6. Procédé selon la revendication 5, dans lequel ledit échantillon biologique est un échantillon de sang, un échantillon de sérum ou un échantillon de plasma.

7. Procédé selon l'une quelconque des revendications 1 à 6, dans lequel ledit cancer du pancréas est un adénocarcinome du pancréas.

8. Utilisation de CXCL4L1 comme biomarqueur du cancer du pancréas chez un patient.

9. Molécule se liant spécifiquement à CXCL4L1 conjugué à un agent anticancéreux tel qu'un agent cytotoxique ou un agent inhibiteur de croissance pour son utilisation dans le traitement du cancer du pancréas et/ou la prévention de métastases pancréatiques, ladite molécule se liant spécifiquement à CXCL4L1 étant un anticorps anti-CXCL4L1 ou un aptamère.

10. Molécule se liant de façon spécifique à CXCL4L1 pour son utilisation dans le traitement du cancer du pancréas et/ou la prévention de métastases pancréatiques selon la revendication 9, ladite molécule se liant spécifiquement à CXCL4L1 étant un anticorps anti-CXCL4L1.
